# EUROPÄISCHE PATENTSCHRIFT

(11) **EP 0 895 469 B1**
(45) Veröffentlichungstag und Bekanntmachung des Hinweises auf die Patenterteilung: **29.03.2006**
(21) Anmeldenummer: 97920645.5
(22) Anmeldetag: 10.04.1997
(51) Int. Cl.: A61K 8/00, A61Q 5/00

(54) **VERFAHREN UND MITTEL ZUM GLEICHZEITIGEN FÄRBEN UND PFLEGEN VON KERATINISCHEN FASERN**
PROCESS AND MEANS FOR SIMULTANEOUSLY COLOURING AND PROTECTING KERATINOUS FIBRES
PROCEDE ET AGENT PERMETTANT SIMULTANEMENT DE COLORER ET DE SOIGNER DES FIBRES DE KERATINE

(30) Priorität: 18.04.1996 DE 19615145
(43) Veröffentlichungstag der Anmeldung: 10.02.1999
(73) Patentinhaber: Henkel Kommanditgesellschaft auf Aktien, 40589 Düsseldorf (DE)
(72) Erfinder: MÜLLER, Reinhard, D-41812 Erkelenz (DE); HÖFFKES, Horst, D-40595 Düsseldorf (DE); HOLLENBERG, Detlef, D-40699 Erkrath (DE); KITTEL, Sabine, D-40721 Hilden (DE)
(86) Internationale Anmeldenummer: PCT/EP1997/001786
(87) Internationale Veröffentlichungsnummer: WO 1997/039725

(56) Entgegenhaltungen:
- EP-A- 0 287 876
- EP-A- 0 535 447
- WO-A-87/03196
- WO-A-92/13829
- WO-A-92/17155
- DE-A- 4 440 315
- DE-C- 4 430 521
- US-A- 4 374 125
- DATABASE WPI Week 9442 Derwent Publications Ltd., London, GB; AN 94-338188 XP002039519 "Hair cosmetic material compsn. having good hair-conditioning and dyeing effects - contains cationic surfactant(s), tar type dye(s) and an amino acid" & JP 06 263 621 A (LION) , 20.September 1994

## Beschreibung

Die Erfindung betrifft ein Verfahren zum gleichzeitigen Färben und Pflegen von keratinischen Fasern sowie Mittel zur Verwendung in diesem Verfahren.

Jede reinigende oder kosmetische Behandlung von Keratinfasern, insbesondere von Haaren, stellt einen Eingriff in die natürliche Struktur der Faser dar. Dies hat zur Folge, daß zum Beispiel Haare nach einer solchen Behandlung, wie sie das Waschen, Färben oder Dauerwellen darstellt, häufig neben den gewünschten Änderungen auch eine Reihe von unbefriedigenden Eigenschaften aufweisen. Besonders im Zusammenhang mit reduzierenden oder oxidierenden Haarbehandlungen ist in diesem Zusammenhang eine verringerte Reißfestigkeit, d.h. eine höhere Auskämmrate, zu nennen.

Man bemüht sich, diesem Mißstand abzuhelfen, indem man entweder die Aggressivität der Haarbehandlungsmittel zu verringern sucht, oder diesen Mitteln Komponenten beigibt, die den unerwünschten Effekten entgegenwirken. Weitere Möglichkeiten sind eine gezielte Vor- und/oder Nachbehandlung mit seperaten Mitteln, die entsprechende Wirkstoffe enthalten. Die DE-A-4430521 offenbart Haatfärbemittel, welche Z-Pyrrolidon-5-carbonsäure als Konditionierend wirkenden Stoff enthalten. Die WO-A-9217155 offenbart Dauerwell mittel, ... welche Pyrrolidonderivate sowie Panthenol derivate zur Verbesserung der Haareigunschaften enthalten.

Es wurde nun überraschenderweise gefunden, daß man den Schwächungen des inneren Zusammenhalts insbesondere von Haaren, die mit Oxidations- oder Reduktionsmitteln behandelt werden, mit speziellen Wirkstoffkombinationen in unerwartet hohem Maße entgegenwirken kann. Dabei haben diese Wirkstoffkombinationen den weiteren Vorteil, daß sie im Fall einer oxidativen Färbebehandlung die Farbnuance praktisch nicht beeinflussen und auch die Viskosität des Mittels nicht in ungewünschter Weise verändern.

Gegenstand der Erfindung ist somit ein Verfahren zum gleichzeitigen Färben und Pflegen von keratinischen Fasern, insbesondere menschlichen Haaren, bei dem ein Mittel auf das Haar aufgebracht wird, das neben einer die Faser färbenden Komponente (F) eine Wirkstoffkombination enthält, die gebildet wird aus
- einer Verbindung (A) der allgemeinen Formel (I), 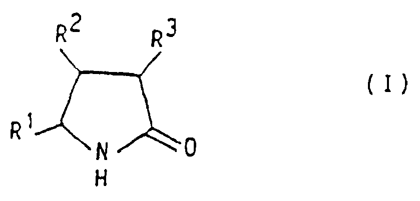 in der mindestens einer der Substituenten R¹ bis R³ steht für eine Gruppe -C00R⁴, in der R⁴ Wasserstoff, ein Alkalimetallion, ein Erdalkalimetallion oder ein Ammoniumion ⁺NHR⁵R⁶R⁷, in dem R⁵ bis R⁷ unabhängig voneinander Wasserstoff, Alkylgruppen mit 1 bis 22 C-Atomen, Hydroxyalkylgruppen mit 1 bis 4 C-Atomen, Alkenylgruppen mit 2 bis 22 C-Atomen, Acylgruppen mit 2 bis 22 C-Atomen oder gegebenenfalls substituierte aromatische Gruppen mit 6 bis 10 C-Atomen sind, ist, und die restlichen Substituenten R¹ bis R³ für Wasserstoff oder Alkylgruppen mit 1 bis 4 C-Atomen stehen, und
- mindestens einer Verbindung (B), ausgewählt aus Panthenol, Panthenol-Ethern, Panthenol-Estern und kationisch derivatisierten Panthenolen.

Die zwingenden Bestandteile der in dem erfindungsgemäßen Verfahren verwendeten Mittel sind in der Kosmetik bekannte Stoffe.

Natürliche, das Haar färbende Stoffe waren bereits im Altertum bekannt; synthetische Haarfarbstoffe sind dem Fachmann seit Jahrzehnten bekannt.

2-Pyrrolidon-5-carbonsäure ist Bestandteil des sogenannten natürlichen Feuchthaltefaktors ("Natural Moisturizing Factor", NMF) der Haut. Diese Verbindung bzw. ihr Natriumsalz wird daher als Feuchthaltemittel für Hautcremes und andere kosmetische Produkte eingesetzt. Weiterhin ist bekannt, daß die Salze die Weichheit und Elastizität der Hornschicht sowie der Haaroberfläche erhöhen (H.P.Fiedler, Lexikon der Hilfsstoffe für Pharmazie, Kosmetik und angrenzende Gebiete, 3. Auflage, Editio Cantor Aulendorf, (1989), S. 110, 111).

Panthenol ist als kosmetischer Wirkstoff beispielsweise aus der deutschen 0ffenlegungsschrift 37 11 841 bekannt.

Aus der PCT-0ffenlegungsschrift W0 92/13829 sind schließlich kationische Panthenolderivate bekannt, die sich durch eine erhöhte Substantivität auf dem Haar auszeichnen und u.a. dem durch Kämmen verursachten Haarspliß entgegenwirken.

Der bekannte Stand der Technik liefert dem Fachmann allerdings keinen Hinweis auf eine Kombination dieser drei Komponenten oder gar die dadurch erhaltenen synergistischen Wirkungen.

Die Verbindungen der Formel (I) sind Derivate des 2-Pyrrolidons.

Bevorzugte Verbindungen sind die 2-Pyrrolidon-3-, -4- und -5-carbonsäure und deren Salze. Bevorzugte Salze dieser Verbindungen sind die Natrium-, Kalium-, Calcium-, Magnesium-, Aluminium- und solche Ammoniumsalze, bei denen das Ammoniumion neben Wasserstoff eine bis drei C₁- bis C₄-Alkyl-oder Hydroxyalkylgruppen trägt.

Besonders bevorzugte Verbindungen der allgemeinen Formel (I) sind die 2-Pyrrolidon-5-carbonsäure und deren Salze. Das Natriumsalz ist ganz besonders bevorzugt.

Als zweite Komponente ist in der erfindungsgemäßen Wirkstoffkombination mindestens eine Verbindung (B), ausgewählt aus Panthenol, Panthenol-Ethern, Panthenol-Estern und kationisch derivatisierten Panthenolen, enthalten.

Erfindungsgemäß verwendbare Panthenol-Ether bzw. Panthenolester sind beispielsweise das Triacetat des Panthenols sowie der Panthenolmonoethylether und dessen Monoacetat.

Bevorzugte Verbindungen (B) sind aber das Panthenol selbst sowie kationische derivatisierte Panthenole.

Bevorzugte kationisch derivatisierte Panthenole sind Verbindungen der Formel (II), 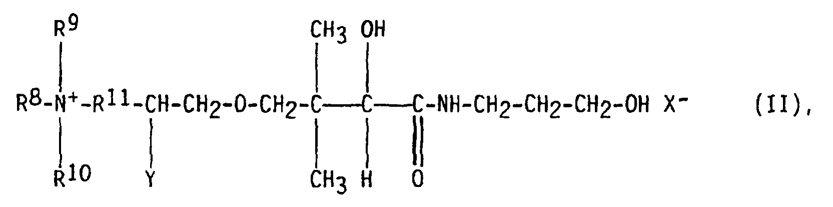 in der R⁸, R⁹ und R¹⁰ unabhängig voneinander für eine Alkylgruppe mit 1-24 Kohlenstoffatomen oder eine Alkenylgruppe mit 8-24 Kohlenstoffatomen, R¹¹ für eine Alkylengruppe mit 1-18 Kohlenstoffatomen, Y für eine OH-Gruppe oder für Wasserstoff und X für ein einwertiges, organisches oder anorganisches Anion steht.

Bevorzugt sind dabei solche Verbindungen gemäß Formel (II), bei denen R⁸ für eine Alkyl- oder Alkenylgruppe mit 8-24 Kohlenstoffatomen steht. Solche Alkyl- bzw. Alkenylgruppen sind beispielsweise Lauryl-, Myristyl-, Cetyl-, Stearyl- und Oleyl-Gruppen. R⁹ und R¹⁰ sind bevorzugt Alkylgruppen mit 1-4 Kohlenstoffatomen, insbesondere Methylgruppen. R¹¹ ist bevorzugt eine kürzere Alkylengruppe mit 1-4 Kohlenstoffatomen, insbesondere eine Methylengruppe. Y steht, herstellungsbedingt, bevorzugt für eine Hydroxygruppe, X für ein Halogenidion, insbesondere für Chlorid.

Informationen bezüglich der Herstellung dieser Panthenol-Derivate sind der genannten Druckschrift WO 92/13829 zu entnehmen, auf die hier ausdrücklich Bezug genommen wird. Ein entsprechendes Produkt wird von der Firma Tri-K unter der Bezeichnung PANTHEQUAT^{R} vertrieben.

Weiterhin enthalten die in dem erfindungsgemäßen Verfahren verwendeten Mittel mindestens einen synthetischen oder natürlichen Farbstoff oder dessen Vorprodukte.

Gemäß einer bevorzugten Ausführungsform enthalten diese Mittel mindestens einen natürlichen Farbstoff. Als natürliche Farbstoffe werden erfindungsgemäß solche Farbstoffe verstanden, die aus Naturprodukten mit üblichen physikalischen Trennverfahren gewonnen und nicht mehr chemisch modifiziert wurden. Solche, im weiteren auch als Naturfarbstoffe bezeichneten Substanzen sind beispielsweise Henna rot, Henna schwarz (Indigofera argentea), Henna neutral, Kamillenblüte, Sandelholz, schwarzer Tee, Faulbaumrinde, Salbei, Blauholz, Krappwurzel, Catechu, Sedre und Alkannawurzel. Bevorzugte natürliche Farbstoffe für die erfindungsgemäßen Mittel sind Henna rot, Henna schwarz, Henna neutral, Kamillenblüte, Sandelholz und schwarzer Tee. Die Naturfarbstoffe sind in den erfindungsgemäßen, auf das Haar aufgebrachten Färbemitteln üblicherweise in Mengen von etwa 5-70 Gew.-%, bevorzugt in Mengen von 8-50 Gew.-%, enthalten.

In einer weiteren bevorzugten Ausführungsform enthalten die erfindungsgemäßen Färbe- und Tönungsmittel als färbende Komponenten ausschließlich Naturfarbstoffe. Es ist jedoch auch möglich, diesen Mitteln zusätzlich noch übliche synthetische Oxidationsfarbstoffvorprodukte, sogenannte Entwickler- und Kupplerkomponenten, und/oder direktziehende Farbstoffe in bevorzugt untergeordneten Mengen zuzugeben.

Als Entwicklerkomponenten können beispielsweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt werden. Spezielle Vertreter sind beispielsweise p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 2-Aminomethyl-1-hydroxy-4-aminobenzol, 1-Phenyl-3-carboxyamido-4-amino-pyrazolon-5 und 4-Amino-3-methylphenol, 2-Hydroxy-4,5,6-triaminopyrimidin, 2,4-Dihydroxy-5,6-diamino-pyrimidin und 2,5,6-Triamino-4-hydroxypyrimidin.

Als Kupplerkomponenten werden bevorzugt m-Phenylendiaminderivate, Naphthole, Resorcin und Resorcinderivate, Pyrazolone und m-Aminophenole verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methylpyrazolon-5, 2,4-Dichlor-3-aminophenol, 1,3-Bis-(2,4-diaminophenoxy)-propan, 2,6-Dihydroxy-3,4-dimethylpyridin, 2-Amino-3-hydroxypyridin, 3-Amino-2-methylamino-6-methoxypyridin, 2,6-Dimethoxy-3,5-diaminopyridin, 4-Chlor-resorcin, 3-Amino-2-chlor-6-methylphenol und 2-Methylresorcin.

Die Entwickler- und Kupplerkomponenten können in der Regel in freier Form eingesetzt werden. Bei Substanzen mit Aminogruppen kann es aber bevorzugt sein, sie in Salzform, insbesondere in Form der Hydrochloride und Hydrobromide, einzusetzen. Weiterhin ist es bevorzugt, solche Oxidationsfarbstoffvorprodukte zu verwenden, die allein aufgrund von Luftoxidation oder bei Anwesenheit geeigneter Enzyme in Kombination mit den entsprechenden Substraten auch in Abwesenheit größerer Konzentrationen von Wasserstoffperoxid zu den entsprechenden Ausfärbungen befähigt sind.

Erfindungsgemäß verwendbare direktziehende Farbstoffe sind beispielsweise Nitrophenylendiamine, Nitroaminophenole, Anthrachinone oder Indophenole, wie die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16, Pikraminsäure und Rodol 9 R bekannten Verbindungen sowie 4-N-Ethyl-1,4-bis((2'-hydroxyethylamino-2-nitro)benzol, 1-(2'-Hydroxyethyl)-amino-4-methyl-2-nitro-benzol, 1-Amino-2-nitro-4(2'hydroxyethylamino)-5-chlorbenzol, 4-Amino-2-nitro-diphenylamin-2'-carbonsäure und 6-Nitro-1,2,3,4-tetrahydrochinoxalin.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die synthetischen Oxidationsfarbstoffvorprodukte bzw. direktziehenden Farbstoffe sind in den erfindungsgemäßen Färbemitteln, sei es als einzige färbende Komponente oder in Kombination mit Naturfarbstoffen, bevorzugt in Mengen von etwa 0,0001 - 5 Gew.-% enthalten.

Die erfindungsgemäßen Zubereitungen enthalten die Verbindungen der Formel (I) bevorzugt in einer Menge von 0,1 bis 5 Gew.-%, insbesondere 0,3 bis 2 Gew-%, bezogen auf die gesamte, auf das Haar aufgebrachte Zubereitung.

Die erfindungsgemäßen Zubereitungen enthalten die Verbindungen (B) ebenfalls bevorzugt in einer Menge von 0,1 bis 5 Gew.-%, insbesondere 0,2 bis 2 Gew-%, bezogen auf die gesamte auf das Haar aufgebrachte Zubereitung.

Gemäß einer bevorzugten Ausführungsform werden in dem erfindungsgemäßen Verfahren bevorzugt solche Mittel eingesetzt, die für eine gewisse Einwirkzeit, die im Bereich von etwa einer Minute bis zu etwa einer Stunde, insbesondere im Bereich von etwa 10 Sekunden bis etwa 40 Minuten, liegt, auf dem Haar verbleiben und anschließend mit Wasser oder einem wäßrigen Mittel ausgespült werden. Unter wäßrigen Mitteln werden erfindungsgemäß solche Mittel verstanden, die im wesentlichen aus Wasser bestehen und weitere Komponenten nur in untergeordneten Mengen, insbesondere in Mengen unterhalb von 20, insbesondere unterhalb von 10, Gew.-% enthalten.

Die in dem erfindungsgemäßen Verfahren auf das Haar aufgebrachten Mittel können in unterschiedlicher Weise konfektioniert und bereitgestellt werden.

Gemäß einer ersten bevorzugten Ausführungsform werden Mittel bereitgestellt, die alle drei zwingenden Komponenten (A), (B) und (F) enthalten. Diese Mittel können direkt auf das Haar aufbracht werden, wenn sie lediglich Naturfarbstoffe und/oder direktziehende Farbstoff und/oder allein durch Luft oxidierbare Oxidationsfarbstoffvorläufer enthalten. Bei der Verwendung üblicher Oxidationsfarbstoffe werden diese Mittel noch unmittelbar vor der Anwendung mit einem Oxidationsmittel, üblicherweise einer wäßrigen Wasserstoffperoxidlösung, gegebenenfalls in Kombination mit Enzymen, vermischt.

Diese Mittel werden üblicherweise auf wäßriger Basis formuliert und können alle in Haarbehandlungsmitteln üblichen Bestandteile enthalten. Dies sind beispielsweise
- anionische Tenside wie Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist, lineare Fettsäuren mit 10 bis 22 C-Atomen (Seifen), Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 10 ist, Acylsarcoside mit 10 bis 18 C-Atomen in der Acylgruppe, Acyltauride mit 10 bis 18 C-Atomen in der Acylgruppe, Acylisethionate mit 10 bis 18 C-Atomen in der Acylgruppe, Sulfobernsteinsäuremono- und dialkylester mit 8 bis 18 C-Atomen in der Alkylgruppe und Sulfobernsteinsäuremono-alkylpolyoxyethylestern mit 8 bis 18 C-Atomen in der Alkylgruppe und 1 bis 6 Oxyethylgruppen und Acylglutamate, jeweils in Form der Natrium-, Kalium- und Ammonium- sowie die Mono-, Di- und Trialkanolammoniumsalze mit 2 oder 3 C-Atomen in der Alkanolgruppe.
- kationische Tenside wie quartäre Ammoniumverbindungen, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Cetylpyridiniumchlorid, kationische Silikonöle, z.B. Dow Corning Q2-7224, Dow Corning 929, General Electric SM-2059, Wacker SLM-55067 sowie Abil^{R}-Quat 3270 und 3272, Alkylamidoamine, z.B. Tego Amid^{R}S 18, sowie sogenannte "Esterquats", z.B. Dehyquart^{R} AU-46, Dehyquart^{R}F-30 und Dehyquart^{R}F-75.
- zwitterionische Tenside wie die sogenannten Betaine, beispielsweise das Kokosalkyl-dimethylammonium-glycinat und das Kokosacylaminopropyldimethylammoniumglycinat
- ampholytische Tensiden, beispielsweise das N-Kokosalkylaminopropionat, das Kokosacylaminoethylaminopropionat und das C₁₂₋₁₈-Acylsarcosin.
- nichtionogene Tenside, beispielsweise Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C₁₂-C₂₂₋Fettsäuremono- und -diester von Anlagerungsprodukten von 1 bis 30 Mol Ethylenoxid an Glycerin, Glycerinmono- und -diester und Sorbitanmono- und -diester von gesättigten und ungesättigten C₈-C₂₂-Fettsäuren und deren Ethylenoxidanlagerungsprodukte, C₈-C₂₂-Alkylmono- und -oligoglycoside und deren ethoxylierte Analoga und Anlagerungsprodukte von 5 bis 60 Mol Ethylenoxid an Ricinusöl und gehärtetes Ricinusöl.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen und tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Vorzugsweise enthalten die erfindungsgemäßen Zubereitungen oberflächenaktive Verbindungen in Mengen von 0,1 bis 50 Gew.-%, bezogen auf die gesamte Zubereitung.

Weitere fakultative Bestandteile der erfindungsgemäßen Mittel sind
- kationische Polymere wie quaternisierte Celluloseether, z.B. Celquat^{R}H 100, Celquat^{R}L 200 und Polymer JR^{R}400, quaternierte Guar-Derivate, z.B. Cosmedia Guar^{R} C-261 und Jaguar^{R} C 13-S, Copolymere des Vinylpyrrolidons mit quaternierten Derivaten des Dialkylaminoacrylats- und -methacrylats, z.B. Gafquat^{R}734, Gafquat^{R}755 und Gafquat^{R} HS100, Copolymerisate des Vinylpyrrolidons mit Uinylimidazoliummethochlorid, z.B. Luviquat^{R}FC 550, polymere Dimethyldiallylammoniumsalze und deren Copolymere mit Estern und Amiden von Acrylsäure und Methacrylsäure, z.B. Merquat^{R}100 und Merquat^{R}550, kationisch derivatisierte Silikonöle, z.B. Dow Corning Q2-7224, Dow Corning 929, SM-2059, SLM-55067 Abil^{R}-Quat 3270 und 7232, sowie polymere Kondensationsharze von Polyolen und Polyaminen, z.B. Polyquart^{R}H 81,
- anionische Polymere wie Vinylacetat/Crotonsäure-Copolymere, z.B. Luviset^{R} CA-66, Vinylpyrrolidon/Vinylacrylat-Copolymere, z.B. Luviflex^{R} VBM-35, Vinylacetat/Butylmaleat/Isobornylacrylat-Copolymere, z.B. Advantage^{R} CP, Methylvinylether/Maleinsäureanhydrid-Copolymere und deren Ester, z.B. Gantrez^{R} ES 225, Acrylsäure/Ethylacrylat/N-tert.Butylacrylamid-Terpolymere, z.B. Ultrahold^{R}8 (BASF), unvernetzte, teilvernetzte und vernetzte Polyacrylsäuren, Polyacrylsäureester, Acrylsäure-Methacrylsäure-Copolymere und Acrylsäure-Acrylamid-Copolymere, wie sie beispielsweise unter den Bezeichnungen Carbopol^{R}, Latekoll^{R}, Pemulen^{R} und Acrysol^{R} im Handel sind,
- amphotere Polymere wie Copolymere aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern, z.B. Amphomer^{R} und Amphomer^{R} LV-71,
- zwitterionische Polymere wie die Copolymeren aus Acrylamidopropyltrimethylammoniumchlorid oder Methacrylamidopropyltrimethylammoniumchlorid und Acrylsäure oder Methacrylsäure bzw. deren Natriumsalzen, Copolymere aus Dimethyldiallylammoniumchlorid und Acrylsäure, z.B. Merquat^{R}280, Polysiloxan-Polyorganobetain-Copolymere sowie zwitterionische Celluloseether,
- nichtionogene Polymere wie Polyvinylpyrrolidone, z.B. Luviskol^{R} K 30 und Luviskol^{R} K 90, Vinylpyrrolidon/Vinylacetat-Copolymere, z.B. Luviskol^{R} VA 64, Luviskol^{R} VA 73 und Luviskol^{R} VA 37, Vinylpyrrolidon/Dimethylaminoethylmethacrylat/Vinylcaprolactam-Terpolymere, z.B. Copolymer VC-713,
- Verdickungsmittel, wie beispielsweise Celluloseether, unvernetzte und mit Polyolen vernetzte Polyacrylsäuren, Agar-Agar, Guar-Gum, Alginate und Xanthan-Gum,
- Proteinhydrolysate, insbesondere Elastin-, Kollagen-, Keratin-, Milcheiweiß-, Sojaprotein- und Weizenproteinhydrolysate, sowie deren Kondensationsprodukte mit Fettsäuren
- Parfümöle,
- Lösungsvermittler, wie Ethanol, Isopropanol, Ethylenglykol, Propylenglykol, Glycerin und Diethylenglykol,
- Farbstoffe,
- Reduktionsmittel wie Thioglykolsäure, Natriumsulfit und Ascorbinsäure,
- Oxidationsmittel wie Kaliumbromat und Wasserstoffperoxid,
- Antischuppenwirkstoffe wie Piroctone Olamine und Zink Omadine,
- Substanzen zur Einstellung des pH-Wertes wie Citronensäure/Natriumcitrat-Puffer,
- Wirkstoffe wie Allantoin, Pflanzenextrakte und Vitamine
- Lichtschutzmittel,
- Konsistenzgeber wie Zuckerester, Polyolester oder Polyolalkylether,
- Fette und Wachse, wie Walrat, Bienenwachs, Montanwachs, Paraffine und Fettalkohole,
- Überfettungsmittel wie polyethoxylierte Lanolinderivate, Lecithinderivate und Fettsäurealkanolamide
- Komplexbildner wie EDTA und Phosphonsäuren,
- Quell- und Penetrationsstoffe wie Glycerin, Propylenglykolmonoethylether, Guanidine und Harnstoff,
- Trübungsmittel wie Latex,
- Perlglanzmittel wie Ethylenglykolmono- und -distearat,
- Treibmittel wie Propan-Butan-Gemische, N₂O, Dimethylether und Luft, sowie
- Antioxidantien.

Gemäß einer zweiten bevorzugten Ausführungsform wird ein erstes Mittel bereitgestellt, das die Komponenten (A) und (B), bevorzugt in einem Massenverhältnis von 1:3 bis 3:1, enthält. Im Rahmen dieser Ausführungsform kann dieses Mittel unmittelbar vor der Anwendung mit einem zweiten Mittel gemischt werden, das die Farbstoffkomponenten enthält. Sofern dieses zweite Mittel nicht bereits vorher mit einem gegebenenfalls benötigten Oxidationsmittel versehen worden ist, kann dieses Oxidationsmittel auch der Mischung aus erstem und zweitem Mittel unmittelbar vor Anwendung zugegeben werden.

Im Rahmen dieser bevorzugten zweiten Ausführungsform kann es vorteilhaft sein, das erste Mittel in Form eines Konzentrates der Komponenten (A) und (B) zu konfektionieren. Dieses Konzentrat besteht dann im wesentlichen aus 5 - 70 Gew.-% einer Verbindungen der Formel (I), 5 - 70 Gew.-% Verbindungen (B) und Wasser. Als weitere Komponenten kommen in erster Linie noch Konservierungsmittel in Betracht. Insbesondere bei Formulierung dieses Mittels als Konzentrat kann dieses, unverdünnt oder mit Wasser verdünnt, im Rahmen dieser bevorzugten Ausführungsform erfindungsgemäß auch unmittelbar vor dem Auftragen einer den oder die Farbstoffe enthaltenden Zubereitung auf das Haar aufgebracht werden. Bei Wahl dieses Anwendungsverfahrens darf allerdings zwischen dem Aufbringen der beiden Zubereitungen auf das Haar keine Zwischenspülung erfolgen.

Gemäß einer dritten bevorzugten Ausführungsform wird ein erstes Mittel bereitgestellt, das die Komponenten (A) und (F) enthält. Dieses Mittel wird unmittelbar vor der Anwendung mit einem zweiten Mittel gemischt, das die Komponente (B) enthält. Bei dem zweiten Mittel kann es sich sowohl um eine Lösung oder Dispersion der Komponente (B) oder um die reine Komponente (B), gegebenenfalls in verkapselter, Form handeln. Die Auswahl einer hochkonzentrierten Konfektionierungsform kann bevorzugt sein. Für den gewünschten Färbe- und Pflegeeffekt spielt es dabei keine Rolle, ob ein gegebenenfalls benötigtes Oxidationsmittel dem ersten Mittel vor oder nach der Zugabe des zweiten Mittels zugegeben wird. Auch im Rahmen dieser bevorzugten Ausführungsform ist es möglich, die die Komponente (B) enthaltende Zubereitung direkt auf das Haar aufzubringen, unmittelbar bevor die die Komponenten (A) und (F) enthaltende Zubereitung appliziert wird.

Die auf das Haar aufgebrachten Zubereitungen weisen bevorzugt einen pH-Wert von etwa 7 bis 10,5 auf; eventuelle Einzelkomponenten, die erst unmittelbar vor der Anwendung vermischt werden, können auch davon abweichende pH-Werte aufweisen, die der Fachmann bevorzugt bezüglich der optimalen Stabilität dieser Mittel auswählen wird.

Bei der Durchführung des erfindungsgemäßen Verfahrens kann sowohl der färbende als auch der pflegende Aspekt im Vordergrund stehen. Im ersten Fall wird man, je nach gewünschter Dauerhaftigkeit der Färbung, mit den entsprechenden Farbstoffen arbeiten. Bei temporären Färbungen, die häufig schon durch einfaches Waschen wieder aus dem Haar entfernt werden können und häufig auch als "Tönungen" bezeichnet werden, wird man neben Naturfarbstoffen bevorzugt sogenannte direktziehende Farbstoffe einsetzen. Dauerhafte Färbungen werden dagegen bevorzugt unter (Mit-)verwendung von 0xidationsfarbstoffen durchgeführt. Hier wird durch das erfindungsgemäße Verfahren die gewünschte Färbung unter gleichzeitiger, bestmöglicher Pflege der Haare erzielt. Im zweiten Fall steht dagegen der pflegende Aspekt im Vordergrund, der mit einer geringen Nuancierung der Haarfarbe verbunden wird, die sich nicht negativ auf die Pflegewirkung auswirkt. Als bevorzugte Ausführungsform einer solchen pflegenden Behandlung kann insbesondere eine leichte Grauabdeckung im Haar genannt werden, die von vielen Verbrauchern dann gewünscht wird, wenn zwar die überwiegende Mehrzahl der Haare noch die bisherige, natürliche Haarfarbe aufweisen, einzelne graue oder weiße Haarsträhnen jedoch nicht mehr zu übersehen sind. Im Falle solcher "pflegender Behandlungen" wird man die verwendeten Mittel bevorzugt ohne Zuckerkomponenten, insbesondere ohne Mono- und Oligosaccharide, formulieren.

### Beispiele

Es wird ein Mittel, bestehend aus den folgenden Zubereitungen, bereitgestellt (sämtliche Mengenangaben sind Gewichtsteile)

### 1.1. Pflegekonzentrat 1

### Komponenten

| | |
|---|---|
| Panthenol¹ | 48,0 |
| Ajidew^{R}N 50² | 48,0 |
| Phenonip^{R 3} | 0,5 |
| Sunset Yellow⁴ | 0,001 |
| Wasser | ad 100 |

| | |
|---|---|
| ¹ 50 % Aktivsubstanz in Wasser | |
| ² DL-2-Pyrrolidon-5-carbonsäure-Natriumsalz (50 % in Wasser; INCI-Bezeichnung: Sodium PCA) (AJINOMOTO) | |
| ³ p-Hydroxybenzoesäuremethylester-p-Hydroxybenzoesäureethylesterp-Hydroxybenzoesäurepropylester-p-Hydroxybenzoesäurebutylester-Phenoxyethanol-Gemisch (INCI-Bezeichnung: Penoxyethanol (and) Methylparaben (and) Propylparaben (and) Butylparaben) (NIPA) | |
| ⁴ 1-(4-Sulfo-1-phenylazo)2-naphthol-6-sulfosäure-Dinatriumsalz 15985) (BASF) | |

### 1.2. Pflegekonzentrat 2

### Komponenten

| | |
|---|---|
| Panthequat^{R 5} | 40,0 |
| Ajidew^{R}N 50 | 40,0 |
| Phenonip^{R} | 0,5 |
| Sunset Yellow | 0,001 |
| Wasser | ad 100 |

| | |
|---|---|
| ⁵ N,N-Dimethyl-N-octadecyl-N[2-hydroxy-4[(3-hydroxypropyl)amino]-2,2-dimethyl-4-oxobutyl]-propyl-ammoniumchlorid (ca. 45 % Aktivsubstanz; INCI-Bezeichnung: Panthenyl-Hydroxypropyl-Steardimonium Chloride) (TRI-K) | |

### 1.3. Pflegekonzentrat 3

### Komponenten

| | |
|---|---|
| Panthenol | 20,0 |
| Panthequat^{R} | 20,0 |
| Ajidew^{R}N 50 | 40,0 |
| Phenonip^{R} | 0,5 |
| Sunset Yellow | 0,001 |
| Wasser | ad 100 |

### 2. Färbecreme

### Komponenten

| | |
|---|---|
| Texapon^{R}N25⁶ | 26,0 |
| C12/14-Fettalkohol | 2,0 |
| Lanette^{R} 0⁷ | 6,5 |
| p-Toluylendiamin | 1,0 |
| Resorcin | 0,2 |
| 2,7-Dihydroxynaphthalin | 0,3 |
| Ammoniumsulfat | 1,0 |
| Ammoniak konz. | 6,0 |
| Parfümöl, Wasser | ad 100 |

| | |
|---|---|
| ⁶ Natriumlaurylethersulfat (ca. 28 % Aktivsubstanz in Wasser; INCI-Bezeichnung: Sodium Laureth Sulfate) (HENKEL) | |
| ⁷ C16/18-Fettalkohol (INCI-Bezeichnung: Cetearyl Alcohol) (HENKEL) | |

### 3. Entwicklerlösung

### Komponenten

| | |
|---|---|
| Texapon^{R}N 25 | 2,0 |
| Ammoniak konz. | 0,65 |
| Wasserstoffperoxid (50 % in Wasser) | 12,0 |
| Acrylsol^{R}33⁸ | 12,0 |
| Turpinal^{R}SL⁹ | 1,5 |
| Wasser | ad 100 |

| | |
|---|---|
| ⁸ Säure enthaltendes, vernetztes Acrylpolymer (28 % Aktivsubstanz in Wasser; INCI-Bezeichnung: Acrylic Copolymer) (ROHM & HAAS) | |
| ⁹ 1-Hydroxyethan-1,1-diphosphonsäure (60 % Aktivsubstanz in Wasser; INCI-Bezeichnung: Etidronic Acid) (HENKEL) | |

Eine Zubereitung, bestehend aus 5 ml Pflegekonzentrat (1, 2 oder 3), 50 g Färbecreme und 50 g Entwicklerlösung wurde auf das Haar aufgebracht. Es resultierte sowohl eine hervorragende Färbung als auch eine exzellente Pflegewirkung auf dem Haar.

## Patentansprüche

1. Verfahren zum gleichzeitigen Färben und Pflegen von keratinischen Fasern, insbesondere menschlichen Haaren, **dadurch gekennzeichnet, daß** ein Mittel auf das Haar aufgebracht wird, das neben einer die Faser färbenden Komponente (F) eine Wirkstoffkombination enthält, die gebildet wird aus
- einer Verbindung (A) der allgemeinen Formel (I), 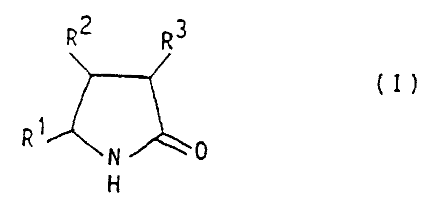 in der mindestens einer der Substituenten R¹ bis R³ steht für eine Gruppe -COOR⁴, in der R⁴ Wasserstoff, ein Alkalimetallion, ein Erdalkalimetallion oder ein Ammoniumion ⁺NHR⁵R⁶R⁷, in dem R⁵ bis R⁷ unabhängig voneinander Wasserstoff, Alkylgruppen mit 1 bis 22 C-Atomen, Hydroxyalkylgruppen mit 1 bis 4 C-Atomen, Alkenylgruppen mit 2 bis 22 C-Atomen, Acylgruppen mit 2 bis 22 C-Atomen oder gegebenenfalls substituierte aromatische Gruppen mit 6 bis 10 C-Atomen sind, ist, und die restlichen Substituenten R¹ bis R³ für Wasserstoff oder Alkylgruppen mit 1 bis 4 C-Atomen stehen, und
- mindestens einer Verbindung (B), ausgewählt aus Panthenol, Panthenolethern, Panthenol-Estern und kationisch derivatisierten Panthenolen.

2. Verfahren nach Anspruch 1, **dadurch gekennzeichnet, daß** das Mittel nach einer Einwirkzeit auf dem Haar, die zwischen 10 Sekunden und 1 Stunde liegt, mit Wasser oder einer wäßrigen Lösung wieder ausgespült wird.

3. Verfahren nach Anspruch 1 oder 2, **dadurch gekennzeichnet, daß** das Mittel stufenweise auf das Haar aufgebracht wird, in dem die Komponenten (A) und (B) als Bestandteile einer ersten Zubereitung ohne Zwischenspülung und unmittelbar vor dem Auftragen einer die Komponente (F) enthaltenden zweiten Zubereitung auf das Haar aufgebracht werden.

4. Mittel zur Verwendung in einem Verfahren nach Anspruch 1 oder 2, **dadurch gekennzeichnet, daß** es
- eine die Faser färbenden Komponente (F) sowie eine Wirkstoffkombination enthält, die gebildet wird aus
- einer Verbindung (A) der allgemeinen Formel (I), in der mindestens einer der Substituenten R¹ bis R³ steht für eine Gruppe -COOR⁴, in der R⁴ Wasserstoff, ein Alkalimetallion, ein Erdalkalimetallion oder ein Ammoniumion ⁺NHR⁵R⁶R⁷, in dem R⁵ bis R⁷ unabhängig voneinander Wasserstoff, Alkylgruppen mit 1 bis 22 C-Atomen, Hydroxyalkylgruppen mit 1 bis 4 C-Atomen, Alkenylgruppen mit 2 bis 22 C-Atomen, Acylgruppen mit 2 bis 22 C-Atomen oder gegebenenfalls substituierte aromatische Gruppen mit 6 bis 10 C-Atomen sind, ist, und die restlichen Substituenten R¹ bis R³ für Wasserstoff oder Alkylgruppen mit 1 bis 4 C-Atomen stehen, und
- mindestens einer Verbindung (B), ausgewählt aus Panthenol, Panthenol-Ethern, Panthenol-Estern und kationisch derivatisierten Panthenolen.

5. Mittel zur Verwendung in einem Verfahren nach einem der Ansprüche 1 bis 3, **dadurch gekennzeichnet, daß** es eine Wirkstoffkombination enthält, die gebildet wird aus
- einer Verbindung (A) der allgemeinen Formel (I), in der mindestens einer der Substituenten R¹ bis R³ steht für eine Gruppe -COOR⁴, in der R⁴ Wasserstoff, ein Alkalimetallion, ein Erdalkalimetallion oder ein Ammoniumion ⁺NHR⁵R⁶R⁷, in dem R⁵ bis R⁷ unabhängig voneinander Wasserstoff, Alkylgruppen mit 1 bis 22 C-Atomen, Hydroxyalkylgruppen mit 1 bis 4 C-Atomen, Alkenylgruppen mit 2 bis 22 C-Atomen, Acylgruppen mit 2 bis 22 C-Atomen oder gegebenenfalls substituierte aromatische Gruppen mit 6 bis 10 C-Atomen sind, ist, und die restlichen Substituenten R¹ bis R³ für Wasserstoff oder Alkylgruppen mit 1 bis 4 C-Atomen stehen, und
- mindestens einer Verbindung (B), ausgewählt aus Panthenol, Panthenol-Ethern, Panthenol-Estern und kationisch derivatisierten Panthenolen.

6. Mittel zur Verwendung in einem Verfahren nach Anspruch 1 oder 2, **dadurch gekennzeichnet, daß** es
- eine die Faser färbenden Komponente (F) und
- eine Verbindung (A) der allgemeinen Formel (I), in der mindestens einer der Substituenten R¹ bis R³ steht für eine Gruppe -COOR⁴, in der R⁴ Wasserstoff, ein Alkalimetallion, ein Erdalkalimetallion oder ein Ammoniumion ⁺NHR⁵R⁶R⁷, in dem R⁵ bis R⁷ unabhängig voneinander Wasserstoff, Alkylgruppen mit 1 bis 22 C-Atomen, Hydroxyalkylgruppen mit 1 bis 4 C-Atomen, Alkenylgruppen mit 2 bis 22 C-Atomen, Acylgruppen mit 2 bis 22 C-Atomen oder gegebenenfalls substituierte aromatische Gruppen mit 6 bis 10 C-Atomen sind, ist, und die restlichen Substituenten R¹ bis R³ für Wasserstoff oder Alkylgruppen mit 1 bis 4 C-Atomen stehen,
enthält.

7. Mittel nach einem der Ansprüche 4 bis 6, **dadurch gekennzeichnet, daß** die Verbindung der allgemeinen Formel (I) 2-Pyrrolidon-5-carbonsäure oder ein Salz dieser Säure ist.

8. Mittel nach Anspruch 7, **dadurch gekennzeichnet, daß** die Verbindung der allgemeinen Formel (I) das Natriumsalz der 2-Pyrrolidon-5-carbonsäure ist.

9. Mittel nach einem der Ansprüche 4 bis 8, **dadurch gekennzeichnet, daß** die Verbindung der Formel (I) in einer Menge von 0,1 bis 5 Gew.-%, insbesondere 0,3 bis 2 Gew-%, bezogen auf die gesamte auf das Haar aufgebrachte Zubereitung, enthalten ist.

10. Mittel nach einem der Ansprüche 4 oder 5, **dadurch gekennzeichnet, daß** die Verbindung (B) ausgewählt ist aus Panthenol sowie kationisch derivatisierten Panthenolen.

11. Mittel nach Anspruch 10, **dadurch gekennzeichnet, daß** als kationisch derivatisiertes Panthenol eine Verbindung der Formel (II) enthalten ist, 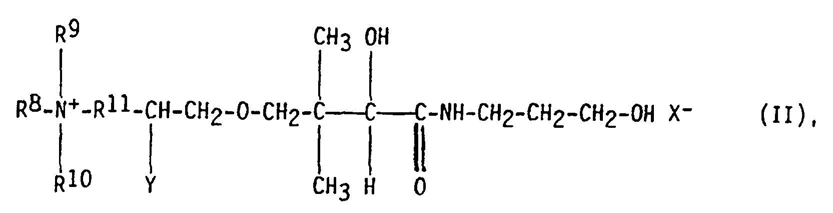 in der R⁸, R⁹ und R10 unabhängig voneinander für eine Alkylgruppe mit 1-24 Kohlenstoffatomen oder eine Alkenylgruppe mit 8-24 Kohlenstoffatomen, R¹¹ für eine Alkylengruppe mit 1-18 Kohlenstoffatomen, Y für eine OH-Gruppe oder für Wasserstoff und X für ein einwertiges, organisches oder anorganisches Anion steht.

12. Mittel nach Anspruch 11, **dadurch gekennzeichnet, daß** das kationisch derivatisierte Panthenol eine Verbindung der Formel (II) ist, in der R⁸ eine Alkyl- oder Alkenylgruppe mit 8-24 Kohlenstoffatomen, R⁹ und R¹⁰ Methylgruppen, und R¹¹ eine Alkylengruppe mit 1-4 Kohlenstoffatomen, Y eine Hydroxygruppe und X ein Halogenidion sind.

13. Mittel nach einem der Ansprüche 4 oder 5, **dadurch gekennzeichnet, daß** die Verbindung (B) in einer Menge von 0,1 bis 5 Gew.-%, insbesondere 0,3 bis 2 Gew-%, bezogen auf die gesamte, auf das Haar aufgebrachte Zubereitung, enthalten ist.

14. Mittel nach Anspruch 4 oder 6, **dadurch gekennzeichnet, daß** es mindestens einen natürlich vorkommenden Farbstoff enthält.

15. Mittel nach Anspruch 14, **dadurch gekennzeichnet, daß** es weiterhin einen direktziehenden Farbstoff oder Oxidationsfarbstoffvorprodukte enthält.

16. Mittel nach Anspruch 5 in Form eines Konzentrats, **dadurch gekennzeichnet, daß** es im wesentlichen besteht aus
- 5 - 70 Gew.-% einer Verbindungen der Formel (I),
- 5 - 70 Gew.-% Verbindungen (B) und
- Wasser.

## Claims

1. Method of simultaneously colouring and caring for keratinous fibres, in particular human hair, **characterized in that** an agent is applied to the hair which, besides a component (F) which colours the fibres, comprises an active ingredient combination which is formed from
- a compound (A) of the general formula (I) 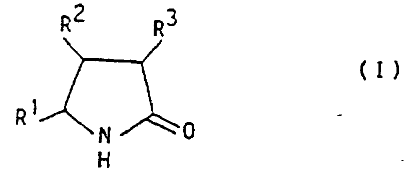
in which at least one of the substituents R¹ to R³ is a group -COOR⁴, in which R⁴ is hydrogen, an alkali metal ion, an alkaline earth metal ion or an ammonium ion ⁺NHR⁵R⁶R⁷, in which R⁵ to R⁷, independently of one another, are hydrogen, alkyl groups having 1 to 22 carbon atoms, hydroxyalkyl groups having 1 to 4 carbon atoms, alkenyl groups having 2 to 22 carbon atoms, acyl groups having 2 to 22 carbon atoms or optionally substituted aromatic groups having 6 to 10 carbon atoms, and the remaining substituents R¹ to R³ are hydrogen or alkyl groups having 1 to 4 carbon atoms, and
- at least one compound (B) chosen from panthenol, panthenol ethers, panthenol esters and cationically derivatized panthenols.

2. Method according to Claim 1, **characterized in that** the agent is rinsed out again after a contact time on the hair which is between 10 seconds and 1 hour, using water or an aqueous solution.

3. Method according to Claim 1 or 2, **characterized in that** the agent is applied to the hair in stages by applying to the hair the components (A) and (B) as constituents of a first preparation without intermediate rinsing and immediately before applying a second preparation comprising the component (F).

4. Agent for use in a method according to Claim 1 or 2, **characterized in that** it comprises
- a component (F) which colours the fibres and an active ingredient combination which is formed from
- a compound (A) of the general formula (I) in which at least one of the substituents R¹ to R³ is a group -COOR⁴, in which R⁴ is hydrogen, an alkali metal ion, an alkaline earth metal ion or an ammonium ion ⁺NHR⁵R⁶R⁷, in which R⁵ to R⁷, independently of one another, are hydrogen, alkyl groups having 1 to 22 carbon atoms, hydroxyalkyl groups having 1 to 4 carbon atoms, alkenyl groups having 2 to 22 carbon atoms, acyl groups having 2 to 22 carbon atoms or optionally substituted aromatic groups having 6 to 10 carbon atoms, and the remaining substituents R¹ to R³ are hydrogen or alkyl groups having 1 to 4 carbon atoms, and
- at least one compound (B) chosen from panthenol, panthenol ethers, panthenol esters and cationically derivatized panthenols.

5. Agent for use in a method according to one of Claims 1 to 3, **characterized in that** it comprises an active ingredient combination which is formed from
- a compound (A) of the general formula (I) in which at least one of the substituents R¹ to R³ is a group -COOR⁴, in which R⁴ is hydrogen, an alkali metal ion, an alkaline earth metal ion or an ammonium ion ⁺NHR⁵R⁶R⁷, in which R⁵ to R⁷, independently of one another, are hydrogen, alkyl groups having 1 to 22 carbon atoms, hydroxyalkyl groups having 1 to 4 carbon atoms, alkenyl groups having 2 to 22 carbon atoms, acyl groups having 2 to 22 carbon atoms or optionally substituted aromatic groups having 6 to 10 carbon atoms, and the remaining substituents R¹ to R³ are hydrogen or alkyl groups having 1 to 4 carbon atoms, and
- at least one compound (B) chosen from panthenol, panthenol ethers, panthenol esters and cationically derivatized panthenols.

6. Agent for use in a method according to Claim 1 or 2, **characterized in that** it comprises
- a component (F) which colours the fibres and
- a compound (A) of the general formula (I) in which at least one of the substituents R¹ to R³ is a group -COOR⁴, in which R⁴ is hydrogen, an alkali metal ion, an alkaline earth metal ion or an ammonium ion ⁺NHR⁵R⁶R⁷, in which R⁵ to R⁷, independently of one another, are hydrogen, alkyl groups having 1 to 22 carbon atoms, hydroxyalkyl groups having 1 to 4 carbon atoms, alkenyl groups having 2 to 22 carbon atoms, acyl groups having 2 to 22 carbon atoms or optionally substituted aromatic groups having 6 to 10 carbon atoms, and the remaining substituents R¹ to R³ are hydrogen or alkyl groups having 1 to 4 carbon atoms.

7. Agent according to one of Claims 4 to 6, **characterized in that** the compound of the general formula (I) is 2-pyrrolidone-5-carboxylic acid or a salt of this acid.

8. Agent according to Claim 7, **characterized in that** the compound of the general formula (I) is the sodium salt of 2-pyrrolidone-5-carboxylic acid.

9. Agent according to one of Claims 4 to 8, **characterized in that** the compound of the formula (I) is present in an amount of from 0.1 to 5% by weight, in particular 0.3 to 2% by weight, based on the total preparation applied to the hair.

10. Agent according to one of Claims 4 and 5, **characterized in that** the compound (B) is chosen from panthenol, and cationically derivatized panthenols.

11. Agent according to Claim 10, **characterized in that** the cationically derivatized panthenol present is a compound of the formula (II) 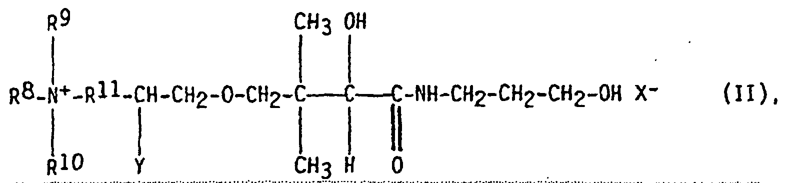 in which R⁸, R⁹ and R¹⁰, independently of one another, are an alkyl group having 1-24 carbon atoms or an alkenyl group having 8-24 carbon atoms, R¹¹ is an alkylene group having 1-18 carbon atoms, Y is an OH group or is hydrogen and X is a monovalent, organic or inorganic anion.

12. Agent according to Claim 11, **characterized in that** the cationically derivatized panthenol is a compound of the formula (II) in which R⁸ is an alkyl or alkenyl group having 8-24 carbon atoms, R⁹ and R¹⁰ are methyl groups, and R¹¹ is an alkylene group having 1-4 carbon atoms, Y is a hydroxy group and X is a halide ion.

13. Agent according to one of Claims 4 and 5, **characterized in that** the compound (B) is present in an amount of from 0.1 to 5% by weight, in particular 0.3 to 2% by weight, based on the total preparation applied to the hair.

14. Agent according to Claim 4 or 6, **characterized in that** it comprises at least one naturally occurring dye.

15. Agent according to Claim 14, **characterized in that** it further comprises a direct dye or oxidation dye precursors.

16. Agent according to Claim 5 in the form of a concentrate, **characterized in that** it essentially consists of
- 5-70% by weight of a compound of the formula (I),
- 5-70% by weight of compounds (B) and
- water.

## Revendications

1. Procédé pour la teinture et le soin simultané de fibres kératiniques, en particulier de cheveux humains, **caractérisé en ce qu'**on applique sur les cheveux un agent qui contient, outre un composant (F) de teinture des cheveux une combinaison de substances actives qui est formée par
- un composé (A) de formule générale (I), 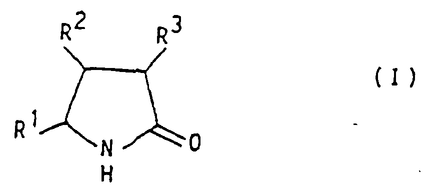
dans laquelle au moins un des substituants R¹ à R³ représente un groupe -COOR⁴, dans lequel R⁴ représente hydrogène, un ion de métal alcalin, un ion de métal alcalino-terreux ou un ion d'ammonium ⁺NHR⁵R⁶R⁷, dans lequel R⁵ à R⁷ représentent, indépendamment l'un de l'autre, hydrogène, des groupes alkyle comprenant 1 à 22 atomes de carbone, des groupes hydroxyalkyle comprenant 1 à 4 atomes de carbone, des groupes alcényle comprenant 2 à 22 atomes de carbone, des groupes acyle comprenant 2 à 22 atomes de carbone ou des groupes aromatiques le cas échéant substitués comprenant 6 à 10 atomes de carbone et les autres substituants R¹ à R³ représentent hydrogène ou des groupes alkyle comprenant 1 à 4 atomes de carbone, et
- au moins un composé (B) choisi parmi le panthénol, les panthénoléthers, les esters de panthénol et les panthénols dérivés cationiquement.

2. Procédé selon la revendication 1, **caractérisé en ce que** l'agent est éliminé par rinçage après un temps d'action sur les cheveux qui est situé entre 10 secondes et 1 heure, avec de l'eau ou une solution aqueuse.

3. Procédé selon la revendication 1 ou 2, **caractérisé en ce que** l'agent est appliqué par étapes sur les cheveux, de manière à ce que les composants (A) et (B) sont appliqués comme constituants d'une première composition, sans rinçage intermédiaire et juste avant l'application d'une deuxième composition contenant le composant (F) sur les cheveux.

4. Agent destiné à être utilisé dans un procédé selon la revendication 1 ou 2, **caractérisé en ce qu'**il contient
- un composant (F) de teinture des fibres ainsi qu'une combinaison de substances actives, formée à partir
- d'un composé (A) de formule générale (I) dans lequel au moins un des substituants R¹ à R³ représente un groupe -COOR⁴, dans lequel R⁴ représente hydrogène, un ion de métal alcalin, un ion de métal alcalino-terreux ou un ion d'ammonium ⁺NHR⁵R⁶R⁷, dans lequel R⁵ à R⁷ représentent, indépendamment l'un de l'autre, hydrogène, des groupes alkyle comprenant 1 à 22 atomes de carbone, des groupes hydroxyalkyle comprenant 1 à 4 atomes de carbone, des groupes alcényle comprenant 2 à 22 atomes de carbone, des groupes acyle comprenant 2 à 22 atomes de carbone ou des groupes aromatiques le cas échéant substitués comprenant 6 à 10 atomes de carbone et les autres substituants R¹ à R³ représentent hydrogène ou des groupes alkyle comprenant 1 à 4 atomes de carbone, et
- d'au moins un composé (B) choisi parmi le panthénol, les panthénoléthers, les esters de panthénol et les panthénols dérivés cationiquement.

5. Agent destiné à être utilisé dans un procédé selon l'une quelconque des revendications 1 à 3, **caractérisé en ce qu'**il contient une combinaison de substances actives qui est formée par
- un composé (A) de formule générale (I) dans lequel au moins un des substituants R¹ à R³ représente un groupe -COOR⁴, dans lequel R⁴ représente hydrogène, un ion de métal alcalin, un ion de métal alcalino-terreux ou un ion d'ammonium ⁺NHR⁵R⁶R⁷, dans lequel R⁵ à R⁷ représentent, indépendamment l'un de l'autre, hydrogène, des groupes alkyle comprenant 1 à 22 atomes de carbone, des groupes hydroxyalkyle comprenant 1 à 4 atomes de carbone, des groupes alcényle comprenant 2 à 22 atomes de carbone, des groupes acyle comprenant 2 à 22 atomes de carbone ou des groupes aromatiques le cas échéant substitués comprenant 6 à 10 atomes de carbone et les autres substituants R¹ à R³ représentent hydrogène ou des groupes alkyle comprenant 1 à 4 atomes de carbone, et
- au moins un composé (B) choisi parmi le panthénol, les panthénoléthers, les, esters de panthénol et les panthénols dérivés cationiquement.

6. Agent destiné à être utilisé dans un procédé selon la revendication 1 ou 2, **caractérisé en ce qu'**il contient
- un composant (F) de teinture des fibres et
- un composé (A) de formule générale (I) dans lequel au moins un des substituants R¹ à R³ représente un groupe -COOR⁴, dans lequel R⁴ représente hydrogène, un ion de métal alcalin, un ion de métal alcalino-terreux ou un ion d'ammonium ⁺NHR⁵R⁶R⁷, dans lequel R⁵ à R⁷ représentent, indépendamment l'un de l'autre, hydrogène, des groupes alkyle comprenant 1 à 22 atomes de carbone, des groupes hydroxyalkyle comprenant 1 à 4 atomes de carbone, des groupes alcényle comprenant 2 à 22 atomes de carbone, des groupes acyle comprenant 2 à 22 atomes de carbone ou des groupes aromatiques le cas échéant substitués comprenant 6 à 10 atomes de carbone et les autres substituants R¹ à R³ représentent hydrogène ou des groupes alkyle comprenant 1 à 4 atomes de carbone.

7. Agent selon l'une quelconque des revendications 4 à 6, **caractérisé en ce que** le composé de formule générale (I) est l'acide 2-pyrrolidone-5-carboxylique ou un sel de cet acide.

8. Agent selon la revendication 7, **caractérisé en ce que** le composé de formule générale (I) est le sel sodique de l'acide 2-pyrrolidone-5-carboxylique.

9. Agent selon l'une quelconque des revendications 4 à 8, **caractérisé en ce que** le composé de formule (I) est contenu en une quantité de 0,1 à 5% en poids, en particulier de 0,3 à 2% en poids, par rapport à la totalité de la préparation appliquée sur les cheveux.

10. Agent selon l'une quelconque des revendications 4 ou 5, **caractérisé en ce que** le composé (B) est choisi parmi le panthénol ainsi que les panthénols dérivés cationiquement.

11. Agent selon la revendication 10, **caractérisé en ce qu'**il contient comme panthénol dérivé cationiquement un composé de formule (II), 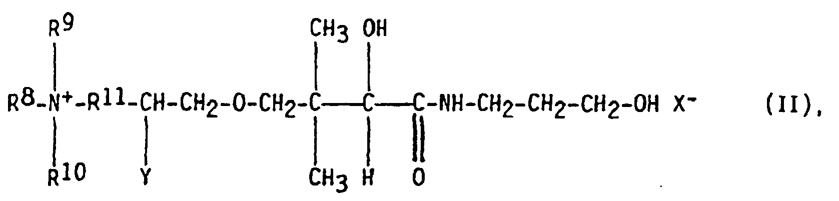 dans laquelle R⁸, R⁹ et R¹⁰ représentent, indépendamment l'un de l'autre, un groupe alkyle comprenant 1 à 24 atomes de carbone ou un groupe alcényle comprenant 8 à 24 atomes de carbone, R¹¹ représente un groupe alkylène comprenant 1 à 18 atomes de carbone, Y représente un groupe OH ou hydrogène et X représente un anion monovalent, organique ou inorganique.

12. Agent selon la revendication 11, **caractérisé en ce que** le panthénol dérivé cationiquement est un composé de formule (II) dans lequel R⁸ représente un groupe alkyle ou alcényle comprenant 8 à 24 atomes de carbone, R⁹ et R¹⁰ représentent des groupes méthyle et R¹¹ représente un groupe alkylène comprenant 1 à 4 atomes de carbone, Y représente un groupe hydroxy et X représente un ion halogénure.

13. Agent selon l'une quelconque des revendications 4 ou 5, **caractérisé en ce que** le composé (B) est contenu en une quantité de 0,1 à 5% en poids, en particulier de 0,3 à 2% en poids, par rapport à la totalité de la composition appliquée sur les cheveux.

14. Agent selon la revendication 4 ou 6, **caractérisé en ce qu'**il contient au moins un colorant naturel.

15. Agent selon la revendication 14, **caractérisé en ce qu'**il contient en outre un colorant montant directement sur la fibre ou des précurseurs d'un colorant d'oxydation.

16. Agent selon la revendication 5 sous forme d'un concentré, **caractérisé en ce qu'**il est essentiellement constitué par
- 5 à 70% en poids d'un composé de formule (I)
- 5 à 70% en poids de composés (B) et
- de l'eau.
